# EUROPÄISCHE PATENTSCHRIFT

(11) **EP 3 110 246 B1**
(45) Veröffentlichungstag und Bekanntmachung des Hinweises auf die Patenterteilung: **11.04.2018**
(21) Anmeldenummer: 15700643.8
(22) Anmeldetag: 05.01.2015
(51) Int. Cl.: A01N 1/02, A61L 27/36

(54) **VERFAHREN ZUR KONSERVIERUNG VON PORCINEM KNORPELGEWEBE**
METHOD FOR PRESERVING PORCINE CARTILAGE TISSUE
PROCÉDÉ POUR CONSERVER DU TISSU CARTILAGINEUX PORCIN

(30) Priorität: 26.02.2014 DE 102014102503
(43) Veröffentlichungstag der Anmeldung: 04.01.2017
(73) Patentinhaber: Tutogen Medical GmbH, 91077 Neunkirchen am Brand (DE)
(72) Erfinder: SCHREINER, Silke, 91080 Uttenreuth (DE)
(74) Vertreter: Manitz Finsterwald Patentanwälte PartmbB
(86) Internationale Anmeldenummer: PCT/EP2015/050027
(87) Internationale Veröffentlichungsnummer: WO 2015/128097

(56) Entgegenhaltungen:
- WO-A1-2012/095316
- WO-A2-2012/141454

## Beschreibung

Die vorliegende Erfindung betrifft ein Verfahren zur Konservierung von porcinem Knorpelgewebe. Gewebetransplantate werden für viele Anwendungen eingesetzt, wie zur Rekonstruktion von Knorpel- und/oder Knochenstrukturen beispielsweise in der Schönheitschirurgie, wie zum Beispiel in der Rhinoplastik. Darüber hinaus werden sie beispielsweise auch bei Operationen an der Wirbelsäule, in der Sportmedizin beispielsweise bei Eingriffen zur Wiederherstellung von Sehnen und Bändern, bei zahnärztlichen Operationen beispielsweise zum Aufbau des Kieferknochens oder zur Weichgeweberegeneration, zum Auffüllen von Knochendefekten im Falle eines Tumors, bei der Korrektur angeborener Defekte, bei der Revision von Hüftprothesen oder bei der Behandlung von Bauchwanddefekten eingesetzt. Dabei können grundsätzlich sowohl Transplantate aus Gewebe natürlichen Ursprungs als auch Transplantate aus synthetischem Material verwendet werden.

Transplantate aus synthetischem Material zeichnen sich durch eine gute Handhabung und Verarbeitbarkeit aus, verheilen jedoch in der Regel schlechter mit dem nativen Gewebe als Transplantate aus Gewebe natürlichen Ursprungs, weswegen letztere bevorzugt sind. Grundsätzlich werden drei Arten von Gewebetransplantaten natürlichen Ursprungs unterschieden, nämlich autogene Gewebetransplantate, bei denen Spender und Empfänger dieselbe Person sind, wobei das Transplantat üblicherweise an einer anderen Stelle als der Entnahmestelle eingesetzt wird, allogene Gewebetransplantate, bei denen Spender und Empfänger verschiedene Individuen einer Spezies sind, sowie xenogene Gewebetransplantate, bei denen der Spender einer anderen Art, beispielsweise Schwein, angehört als der Empfänger, beispielsweise Mensch. Aufgrund der begrenzten Verfügbarkeit allogener und insbesondere autogener Gewebetransplantate sind xenogene Gewebetransplantate von besonderem Interesse. Dabei sind insbesondere Gewebetransplantate aus Schweinen begehrt, weil diese physiologisch besser den Anforderungen an den menschlichen Körper genügen als beispielsweise Gewebetransplantate aus Primaten. Hinzu kommt die nahezu unbegrenzte Verfügbarkeit von porcinen Geweben.

Ein wesentlicher Aspekt bei der Herstellung von Gewebetransplantaten ist es, dass die native Struktur des Gewebes in dem fertigen Transplantat möglichst beibehalten wird, damit dieses beispielsweise im Hinblick auf die innere Oberfläche, die Handhabbarkeit und die Elastizität zumindest weitgehend die Eigenschaften des Nativgewebes aufweist. Während dies bei bekannten Verfahren für einige Gewebe, wie Knochen oder Hohlgewebe, beispielsweise Blutgefäße, erreicht wird, gelingt dies für Knorpel nicht oder zumindest nicht in einem befriedigenden Ausmaß. Bei Knorpel handelt es sich im Vergleich zu anderen Geweben, wie Knochen oder Hohlgeweben, um ein besonderes Gewebe, das sowohl druck- als auch biegungselastisch ist, wobei sich speziell hyaliner Knorpel zudem durch ein hohes Wasseraufnahmevermögen auszeichnet. Knorpel besteht aus in Interzellularsubstanz eingebetteten Knorpelzellen, wobei die Interzellularsubstanz im Wesentlichen aus Kollagenfasern, Proteoglykanen, Hyaluronsäure und Wasser zusammengesetzt ist. Als einziges Gewebe ist Knorpel nicht durchblutet, so dass die Zuführung von Nährstoffen in das Gewebe und die Abführung von Stoffwechselprodukten aus dem Gewebe ausschließlich durch Diffusion erfolgt. Wichtige Beispiele für Knorpel sind beispielsweise hyaline Knorpel, wie Gelenk-, Rippen- und Nasenknorpel, Faserknorpel, wie Meniskus oder Labrum glenoidale des Schultergelenks, und elastischer Knorpel, wie Kehldeckel oder Ohrtrompete. Knorpel unterscheidet sich damit fundamental von anderen Geweben und insbesondere von anderen in der Transplantation eingesetzten Geweben, wie beispielsweise Knochen und Blutgefäßen, welche allesamt durchblutet sind. Zudem unterscheiden sich Knorpel von Blutgefäßen auch darin, dass Knorpel eine sehr viel geringere Zelldichte als Blutgefäßen aufweisen. Ein weiterer Unterschied zwischen Knochen und Knorpel ist, dass auf dem in Knochen enthaltendem Kollagen das Mineral Hydroxylapatit abgelagert ist.

Aus der WO 2012/141454 A2 ist ein Verfahren zum Herstellen eines Knorpeltransplantats bekannt, welches die Schritte des Bereitstellens von Knorpel, Behandeln des Knorpels mit einer hypertonischen Natriumchloridlösung, Behandeln des so erhaltenen Knorpels mit einer Virusinaktivierungslösung, daran anschließendes Behandeln des Knorpels mit einer Alkalilösung und dann Entwässern des Knorpels beispielsweise durch Eintauchen desselben in Alkohol oder Aceton umfasst.

In der WO 2012/095316 A1 wird ein Verfahren zum Herstellen eines Transplantats aus tierischer Dermis offenbart, welches die folgenden Schritte umfasst: Behandeln der Dermis mit Natriumsulfid enthaltender wässriger Lösung, daran anschließend mit einer Salzlösung, daran anschließend mit Wasserstoffperoxid und dann Dehydrieren der Dermis.

Ein anderes bekanntes Verfahren zur Aufbereitung von u.a. Knorpelgewebe zur Verwendung als allogenes oder xenogenes Transplantat ist aus der WO 2010/108945 A1 bekannt. Bei diesem Verfahren wird eine entsprechende Gewebeprobe zunächst einer Behandlung mit einem alkalischen Medium mit einem pH-Wert von mehr als 12, wie insbesondere Natronlauge, unterworfen, bevor die so behandelte Probe mit einem starken chaotropen Salz, insbesondere Guanidiumhydrochlorid, behandelt und dann getrocknet wird. Dabei sollen durch die alkalische Behandlung Kontaminanten, wie Proteine und Lipide, größtenteils hydrolysiert werden, Pathogene, wie Viren und Bakterien, inaktiviert werden und zudem eine Quellung des Gewebes erreicht werden, was die Wirksamkeit der in den nachfolgenden Schritten eingesetzten Chemikalien erhöhen soll. Außerdem sollen durch die nachfolgende Behandlung mit einem starken chaotropen Salz nichtkollagene Bestandteile des Gewebes denaturiert werden. Allerdings führt der alkalische Behandlungsschritt zu irreversiblen Schäden an der nativen Struktur des Knorpelgewebes, so dass die mit diesem Verfahren erhaltenen Knorpelgewebe als unbefriedigend empfunden werden.

Ausgehend davon liegt der vorliegenden Erfindung die Aufgabe zugrunde, ein Verfahren zur Konservierung von tierischem Knorpelgewebe bereitzustellen, welches einfach, schnell und kostengünstig durchgeführt werden kann und zu einem Knorpelgewebetransplantat führt, dessen Morphologie der nativen Struktur des Ausgangsgewebes zumindest weitgehend entspricht. Zudem soll dieses Verfahren insbesondere zur Konservierung von porcinem Knorpelgewebe geeignet sein.

Erfindungsgemäß wird diese Aufgabe durch ein Verfahren zur Konservierung von tierischem Knorpelgewebe, insbesondere von porcinem Knorpelgewebe, gelöst, welches die nachfolgenden Schritte umfasst:
a) Bereitstellen von tierischem Knorpelgewebe,
b) ein- oder mehrmaliges Behandeln des in dem Schritt a) bereitgestellten tierisches Knorpelgewebes mit einer in Bezug auf die Knorpelgewebezellen hypertonen Lösung von einem oder mehreren Alkalimetallhalogenidsalzen,
c) ein- oder mehrmaliges Behandeln des gemäß dem Schritt b) behandelten tierischen Knorpelgewebes mit einem Oxidationsmittel ausgewählt aus der Gruppe bestehend aus Peroxiden, Hypochloriten, Percarbonsäuren, Ozon und beliebigen Mischungen hiervon sowie
d) ein- oder mehrmaliges Behandeln des gemäß dem Schritt c) behandelten tierischen Knorpelgewebes mit einem Lösungsmittel, welches mit Wasser bei 20°C vollständig mischbar ist,
wobei das tierische Knorpelgewebe während des Verfahrens nicht mit einem alkalischen Medium mit einem pH-Wert von 9 oder mehr in Kontakt gebracht wird, und wobei in dem Schritt a) porcines Knorpelgewebe bereitgestellt wird, welches aus einem Schwein mit einem Alter von maximal 2 Jahren stammt.

Diese Lösung beruht auf der überraschenden Erkenntnis, dass durch die Behandlung von tierischem Knorpelgewebe mit einer hypertonen Lösung von einem oder mehreren Alkalimetallhalogenidsalzen, bevorzugt wässriger Natriumchloridlösung, durch nachfolgendes Behandeln mit einem spezifischen Oxidationsmittel, bevorzugt Wasserstoffperoxid, und durch anschließendes Trocknen des so erhaltenen Gewebes mit einem mit Wasser bei 20°C vollständig mischbaren Lösungsmittel, bevorzugt Aceton, eine hervorragende Konservierung von Knorpelgewebe erreicht wird, wobei eine Schädigung der Kollagenmatrix des Knorpelgewebe vollständig oder zumindest nahezu vollständig verhindert wird, so dass ein Knorpelgewebetransplantat erhalten wird, dessen Morphologie der nativen Struktur des Ausgangsgewebes zumindest weitgehend entspricht. Insbesondere wird mit diesem Verfahren ein konserviertes Knorpelgewebe erhalten, welches keine lebenden Knorpelzellen mehr enthält, welches einfach schneidbar ist sowie keine Risse und Unebenheiten aufweist. Dabei werden die Knorpelzellen in dem Verfahrensschritt b) durch die Behandlung mit einer - in Bezug auf die Knorpelgewebezellen - hypertonen Lösung von einem oder mehreren Alkalimetallhalogenidsalzen zum Platzen gebracht, so dass die intrazellulären Bestandteile aus den Zellen herausgewaschen und entfernt werden können, ohne aber die Struktur des Knorpelgewebes und insbesondere des Kollagens zu beeinträchtigen. Die nachfolgende oxidative Behandlung in dem Verfahrensschritt c) führt zu einer Inaktivierung von in dem Gewebe enthaltenen Pathogenen und zu einer Denaturierung wasserlöslicher Antigene, wiederum aber nicht zu einer Beeinträchtigung der Struktur des Knorpelgewebes und insbesondere des Kollagens. Schließlich führt die Behandlung mit vollständig mit Wasser mischbarem Lösungsmittel in dem Verfahrensschritt d) zu einer schonenden, die native Struktur des Knorpelgewebes bewahrenden Entwässerung bzw. Trocknung des Gewebes. Auf diese Weise kann schnell, einfach und kostengünstig tierisches Knorpelgewebe konserviert und tierisches Knorpelgewebetransplantat erhalten werden, dessen Morphologie der nativen Struktur des Ausgangsgewebes zumindest weitgehend entspricht. Dabei ist dieses Verfahren insbesondere zur Konservierung von porcinem Knorpelgewebe geeignet. Entscheidend ist dabei, dass aufgrund der Verfahrensschritte b) bis d) und insbesondere aufgrund der Behandlung des Knorpelgewebes in dem Verfahrensschritt b) mit einer in Bezug auf die Knorpelgewebezellen hypertonen Lösung von einem oder mehreren Alkalimetallhalogenidsalzen auf alkalische Behandlungsschritte und auch auf Behandlungsschritte mit stark chaotropen Substanzen, wie Guanidiumhydrochlorid, welche die Gewebestruktur verändern, verzichtet werden kann.

Wie dargelegt, eignet sich das erfindungsgemäße Verfahren zur Konservierung von allen tierischen Knorpelgeweben und insbesondere zur Konservierung von allen porcinen Knorpelgeweben. Aufgrund der Tatsache, dass Knorpelgewebetransplantate aus Schweinen physiologisch den Anforderungen an den menschlichen Körper besonders gut genügen, wird in dem Verfahrensschritt a) bevorzugt porcines Knorpelgewebe und bevorzugt porcines Rippenknorpelgewebe bereitgestellt.

Erfindungsgemäß wird in dem Verfahrensschritt a) porcines Knorpelgewebe bereitgestellt, welches aus einem Schwein mit einem Alter von maximal 2 Jahren stammt. Überraschenderweise hat sich im Rahmen der vorliegenden Erfindung herausgestellt, dass insbesondere Knorpelgewebe derart junger Schweine zu Knorpelgewebetransplantat mit besonders guten Eigenschaften führt. Ohne an eine Theorie gebunden werden zu wollen, wird es erachtet, dass dies darauf zurückzuführen ist, dass das Knorpelgewebe älterer Schweine oder älterer anderer Tiere bereits zu einem gewissen Ausmaß verkalkt ist, was in Bezug auf die Eigenschaften des mit dem Verfahren hergestellten Transplantats nachteilig ist.

Grundsätzlich kann das tierische Knorpelgewebe in dem Verfahrensschritt b) mit einer beliebigen Lösung von einem beliebigen Alkalimetallhalogenid oder mit einer beliebigen Lösung von einer Mischung aus mehreren beliebigen Alkalimetallhalogeniden behandelt werden, sofern das Lösungsmittel die Struktur des Knorpelgewebes nicht oder zumindest nicht merklich angreift. Gute Ergebnisse werden dabei insbesondere erzielt, wenn das tierische Knorpelgewebe in dem Verfahrensschritt b) mit einer wässrigen Lösung von einem beliebigen Alkalimetallhalogenid oder mit einer wässrigen Lösung von einer Mischung aus mehreren beliebigen Alkalimetallhalogeniden behandelt wird. Dabei besteht die Lösung und bevorzugt die wässrige Lösung vorzugsweise aus dem Lösungsmittel und dem bzw. den Alkalimetallhalogenid(en), enthält also keine weitere Verbindung(en). Dabei weist die in dem Verfahrensschritt b) eingesetzte Lösung einen pH-Wert von unter 9, besonders bevorzugt einen pH-Wert von maximal 8 und ganz besonders bevorzugt einen pH-Wert von maximal 7,5 auf.

Dabei wird als Lösungsmittel mit Vorteil Wasser mit einem hohen Reinheitsgrad eingesetzt und besonders bevorzugt destilliertes Wasser oder Wasser mit einem destilliertem Wasser entsprechenden Reinheitsgrad.

Als Alkalimetallhalogenid kann dabei jedes beliebige eingesetzt werden, also Lithiumchlorid, Natriumchlorid, Kaliumchlorid, Rubidiumchlorid, Cäsiumchlorid und/oder Franciumchlorid, wobei gute Ergebnisse jedoch insbesondere mit einem oder mehreren Alkalimetallhalogeniden ausgewählt aus Lithiumchlorid, Natriumchlorid, Kaliumchlorid und beliebigen Mischungen von zwei oder mehr hiervon erzielt werden.

Ganz besonders bevorzugt wird in dem Verfahrensschritt b) eine Natriumchloridlösung und höchst bevorzugt eine wässrige Natriumchloridlösung eingesetzt. Prinzipiell kann die in dem Verfahrensschritt b) eingesetzte Salzlösung jede Salzkonzentration aufweisen, solange die Lösung in Bezug auf die Knorpelgewebezellen hyperton ist. Dabei sollte die Konzentration jedoch so hoch gewählt werden, dass ein Platzen der Zellen in der Zeit, für welche der Verfahrensschritt durchgeführt wird, zuverlässig erreicht wird, oder aber so niedrig, dass die Struktur und Morphologie des Knorpelgewebes nicht oder zumindest nicht wesentlich beeinträchtigt wird. Gute Ergebnisse werden diesbezüglich erzielt, wenn das tierische Knorpelgewebe in dem Verfahrensschritt b) mit einer wässrigen Lösung behandelt wird, welche 5 bis 20 Gew.-%, bevorzugt 5 bis 15 Gew.-%, besonders bevorzugt 8 bis 12 Gew.-% und höchst bevorzugt etwa 10 Gew.-% eines Alkalimetallhalogenids oder einer Mischung aus zwei oder mehr verschiedener Alkalimetallhalogenid enthält. Vorzugsweise wird in dem Verfahrensschritt b) eine wässrige Lösung eingesetzt, welche 5 bis 20 Gew.-%, bevorzugt 5 bis 15 Gew.-% und besonders bevorzugt 8 bis 12 Gew.-% Natriumchlorid enthält. Ferner ist es bevorzugt, dass die eingesetzte Lösung neben dem eingesetzten Lösungsmittel und darin enthaltenen Alkalimetallhalogenid, insbesondere Natriumchlorid, kein weiteres Salz und auch keine sonstigen Verbindungen enthält und vorzugsweise einen pH-Wert von maximal 9, besonders bevorzugt einen pH-Wert von maximal 8 und ganz besonders bevorzugt einen pH-Wert von maximal 7,5 aufweist.

Um eine besonders vollständige Entfernung der intrazellulären Bestandteile aus dem eingesetzten Knorpelgewebe zu erreichen, wird es in Weiterbildung des Erfindungsgedankens vorgeschlagen, das tierische Knorpelgewebe in dem Verfahrensschritt b) 1- bis 20-mal, bevorzugt 3- bis 15-mal, besonders bevorzugt 5- bis 10-mal, ganz besonders bevorzugt 7- bis 9-mal und höchst bevorzugt 8-mal mit jeweils frischer hypertoner Salzlösung zu behandeln. Zwischen den einzelnen Teilschritten kann das Knorpelgewebe optional ein oder mehrmals mit Wasser oder einem anderen hypotonen Medium gespült werden. Durch eine solche Wechselbadbehandlung mit hypertoner Salzlösung/Wasser bzw. hypertoner Lösung wird der Transport von zellulären Bestandteilen aus der Gewebestruktur durch Diffusionsvorgänge infolge wechselnder osmotischer Verhältnisse in dem Gewebe begünstigt und somit der Effekt der Salzbehandlung verbessert.

Gute Ergebnisse werden dabei insbesondere erzielt, wenn jede Behandlung in dem Verfahrensschritt b) für 1 bis 48 Stunden, bevorzugt für 5 bis 36 Stunden, besonders bevorzugt für 12 bis 24 Stunden und ganz besonders bevorzugt für 14 bis 20 Stunden durchgeführt wird, und zwar bevorzugt mit einem, bezogen auf das Gewicht des tierischen Knorpelgewebes, 5- bis 15-fachen Volumen, bevorzugt 6-bis 14-fachen Volumen, insbesondere bevorzugt 7- bis 13-fachen Volumen, weiter bevorzugt 8- bis 12-fachen Volumen und ganz besonders bevorzugt 9- bis 11-fachen, wie insbesondere 10-fachen, Volumen an hypertoner Salzlösung.

Dabei kann der Verfahrensschritt b) optional beispielsweise auf einer Rüttelvorrichtung oder in einer rotierenden Trommel durchgeführt werden. Optional kann die in dem Verfahrensschritt b) eingesetzte Lösung mit Ultraschall beaufschlagt werden.

In dem Verfahrensschritt c) wird das zuvor mit Salzlösung behandelte Knorpelgewebe mit einem Oxidationsmittel ausgewählt aus der Gruppe bestehend aus Peroxiden, Hypochloriten, Percarbonsäuren, Ozon und beliebigen Mischungen hiervon behandelt, um in dem Gewebe enthaltene Pathogene zu inaktivieren und wasserlösliche Antigene zu denaturieren. Vorzugsweise werden dabei wässrige Lösungen ein oder mehrerer diese Substanzen eingesetzt. Dabei beträgt die Konzentration des Oxidationsmittels oder, im Falle des Einsatzes eine Mischung aus zwei oder mehr Oxidationsmittel, die Summe der Konzentrationen der Oxidationsmittel vorzugsweise 0,5 bis 10 Gew..-%, besonders bevorzugt 1 bis 5 Gew.-% und ganz besonders bevorzugt 2 bis 4 Gew.-%, wie insbesondere bevorzugt etwa 3 Gew.-%. Ferner ist es bevorzugt, dass die eingesetzte Lösung neben dem eingesetzten Lösungsmittel und dem darin enthaltenen Oxidationsmittel keine weiteren Verbindungen enthält und vorzugsweise einen pH-Wert von maximal 9, besonders bevorzugt einen pH-Wert von maximal 8 und ganz besonders bevorzugt einen pH-Wert von maximal 7,5 aufweist.

Gemäß einer besonders bevorzugten Ausführungsform der vorliegenden Erfindung wird in dem Verfahrensschritt c) eine wässrige Wasserstoffperoxidlösung eingesetzt, welche 0,5 bis 10 Gew.-%, bevorzugt 1 bis 5 Gew.-% und besonders bevorzugt 2 bis 4 Gew.-%, wie insbesondere 3 Gew.-%, Wasserstoffperoxid enthält. Dabei wird als Lösungsmittel mit Vorteil Wasser mit einem hohen Reinheitsgrad und besonders bevorzugt destilliertes Wasser oder Wasser mit einem destillierten Wasser entsprechenden Reinheitsgrad eingesetzt.

Um eine besonders vollständige Inaktivierung von in dem Gewebe enthaltenen Pathogenen und eine besonders vollständige Denaturierung wasserlöslicher Antigene zu erreichen, wird es in Weiterbildung des Erfindungsgedankens vorgeschlagen, das tierische Knorpelgewebe in dem Verfahrensschritt c) 1- bis 5-mal, bevorzugt 1- bis 3-mal und besonders bevorzugt 2-mal mit jeweils frischem Oxidationsmittel und bevorzugt mit frischer wässriger Wasserstoffperoxidlösung zu behandeln. Zwischen den einzelnen Teilschritten kann das Knorpelgewebe optional ein oder mehrmals mit Wasser, Oxidationsmittel oder physiologischer Kochsalzlösung gespült werden.

Gute Ergebnisse werden dabei insbesondere erzielt, wenn jede Behandlung in dem Verfahrensschritt c) für 1 bis 48 Stunden, bevorzugt für 5 bis 36 Stunden, besonders bevorzugt für 12 bis 24 Stunden und ganz besonders bevorzugt für 14 bis 20 Stunden durchgeführt wird, und zwar bevorzugt mit einem, bezogen auf das des tierischen Knorpelgewebes, 5- bis 15-fachen Volumen, bevorzugt 6- bis 14-fachen Volumen, insbesondere bevorzugt 7- bis 13-fachen Volumen, weiter bevorzugt 8- bis 12-fachen Volumen und ganz besonders bevorzugt 9- bis 11-fachen, wie insbesondere 10-fachen, Volumen an Oxidationsmittel und bevorzugt wässriger Wasserstoffperoxidlösung durchgeführt wird.

Dabei kann der Verfahrensschritt b) optional beispielsweise auf einer Rüttelvorrichtung oder in einer rotierenden Trommel durchgeführt werden. Optional kann die in dem Verfahrensschritt b) eingesetzte Lösung mit Ultraschall beaufschlagt werden.

Gemäß einer weiteren besonders bevorzugten Ausführungsform der vorliegenden Erfindung wird das tierische Knorpelgewebe in dem Verfahrensschritt d) mit einem Lösungsmittel behandelt, welches aus der Gruppe ausgewählt wird, welche aus Aceton, Methanol, Ethanol, Propanol, Isopropanol, Methylethylketon und beliebigen Mischungen aus zwei oder mehr dieser Verbindungen besteht. Durch diese Lösungsmittel wird in dem Verfahrensschritt d) eine besonders schonende Entwässerung bzw. Trocknung des Knorpelgewebes erreicht, und zwar unter Beibehaltung oder zumindest nahezu vollständiger Beibehaltung der nativen Struktur des Knorpelgewebes. Besonders gute Ergebnisse werden dabei insbesondere erreicht, wenn in dem Verfahrensschritt d) als Lösungsmittel Aceton eingesetzt wird. Vorzugsweise wir reines Lösungsmittel eingesetzt, d.h. Lösungsmittel, welche keine weiteren Verbindungen enthält.

Um eine wirksame Entwässerung bzw. Trocknung sicherzustellen, ist es gemäß einer weiteren besonders bevorzugten Ausführungsform der vorliegenden Erfindung vorgesehen, dass das tierische Knorpelgewebe in dem Verfahrensschritt d) 1- bis 15-mal, bevorzugt 3- bis 10-mal, besonders bevorzugt 4- bis 9-mal, ganz besonders bevorzugt 6- bis 8-mal und höchst bevorzugt 7-mal mit jeweils frischem Lösungsmittel, insbesondere Aceton, behandelt wird.

Dabei wird mit Vorteil jede Behandlung in dem Verfahrensschritt d) für 1 bis 48 Stunden, bevorzugt für 5 bis 36 Stunden, besonders bevorzugt für 12 bis 24 Stunden und ganz besonders bevorzugt für 14 bis 20 Stunden durchgeführt, und zwar bevorzugt mit einem, bezogen auf das des tierischen Knorpelgewebes, 5- bis 15-fachen Volumen, bevorzugt 6- bis 14-fachen Volumen, insbesondere bevorzugt 7-bis 13-fachen Volumen, weiter bevorzugt 8- bis 12-fachen Volumen und ganz besonders bevorzugt 9- bis 11-fachen, wie insbesondere 10-fachen, Volumen Lösungsmittel.

Dabei kann der Verfahrensschritt b) optional beispielsweise auf einer Rüttelvorrichtung oder in einer rotierenden Trommel durchgeführt werden. Optional kann die in dem Verfahrensschritt b) eingesetzte Lösung mit Ultraschall beaufschlagt werden.

Gemäß einer ganz besonders bevorzugten Ausführungsform der vorliegenden Erfindung wird nach der Trocknung von dem Knorpel in einem Verfahrensschritt e) die Knorpelhaut bzw. das Perichondrium entfernt, wonach der Knorpel ferner optional auf die gewünschten Ausmaße geschnitten wird. Dabei wird die Entfernung des Perichondriums bevorzugt mechanisch durchgeführt, und zwar besonders bevorzugt durch Fräsen, wie beispielsweise mit Hilfe eines Dremels.

Anschließend wird das Knorpelgewebe bevorzugt in einer wässrigen Lösung und besonders bevorzugt in einer wässrigen physiologischen Kochsalzlösung rehydratisiert und gelagert.

Anschließend kann der in Flüssigkeit gelagerten Knorpel verpackt und bevorzugt sterilisiert werden. Dabei wird die Sterilisierung vorzugsweise durch Bestrahlung erreicht, und zwar beispielsweise durch Bestrahlung mit gamma-Strahlen mit einer Dosis von 1 bis 100 kGy, bevorzugt 5 bis 50 kGy, besonders bevorzugt 10 bis 30 kGy und höchst bevorzugt etwa 20 kGy.

Wie vorstehend dargelegt, ist das erfindungsgemäße Verfahren zur Konservierung von tierischem Knorpelgewebe dadurch gekennzeichnet, dass das tierische Knorpelgewebe während des Verfahrens nicht mit einem alkalischen Medium mit einem pH-Wert von 9 oder mehr in Kontakt gebracht wird. Dabei ist es bevorzugt, dass das Knorpelgewebe während des Verfahrens nicht mit einem alkalischen Medium mit einem pH-Wert von 8 oder mehr in Kontakt gebracht wird. Dadurch kann eine Beeinträchtigung der nativen Morphologie und Struktur des eingesetzten Knorpelgewebes zuverlässig verhindert werden.

Gemäß einer ganz besonders bevorzugten Ausführungsform der vorliegenden Erfindung umfasst das erfindungsgemäße Verfahren die nachfolgenden Schritte:
a) Bereitstellen von porcinem Knorpelgewebe, welches aus einem Schwein mit einem Alter von maximal 2 Jahren stammt,
b) ein- oder mehrmaliges Behandeln des porcinem Knorpelgewebes mit einer wässrigen Lösung, welche 5 bis 20 Gew.-%, bevorzugt 5 bis 15 Gew.-% und besonders bevorzugt 8 bis 12 Gew.-% Natriumchlorid enthält,
c) ein- oder mehrmaliges Behandeln des tierisches Knorpelgewebes mit einer 1 bis 5 Gew.-% Wasserstoffperoxid enthaltenden wässrigen Lösung und
d) ein- oder mehrmaliges Behandeln des tierisches Knorpelgewebes mit Aceton,
wobei das tierische Knorpelgewebe während des Verfahrens nicht mit einem alkalischen Medium mit einem pH-Wert von 9 oder mehr und besonders bevorzugt nicht mit einem alkalischen Medium mit einem pH-Wert von 8 oder mehr in Kontakt gebracht wird. Dadurch kann eine Beeinträchtigung der nativen Morphologie und Struktur des eingesetzten Knorpelgewebes zuverlässig verhindert werden.

Nachfolgend wird die vorliegende Erfindung anhand eines Ausführungsbeispiels näher erläutert.

### Beispiel

Aus einem frisch geschlachteten Schwein mit einem Alter von 0,5 Jahren wurde Rippenknorpel isoliert und etwaig daran haftendes Fett und dieses umgebendes Gewebe entfernt. Der Knorpel wurde in Stücke mit Ausmaßen von jeweils 6 cm x 1 cm x 1 cm geschnitten.

Danach wurden porcine Rippenknorpelstücke in einer Trommel gemäß dem nachfolgenden Behandlungsschema behandelt:

| **Schritt** | **Aktion** | **Zeit (h)** |
|---|---|---|
| 1 | Spülen mit Wasser (3x) | |
| 2 | Behandeln mit 10-fachem Volumen wässriger 10 Gew.-% Natriumchloridlösung | 20 Std. |
| 3 | Spülen mit H₂O (3x) | Je 5 Min. |
| 4 | Behandeln mit 10-fachem Volumen wässriger 10 Gew.-% Natriumchloridlösung | 20 Std. |
| 5 | Spülen mit H₂O (3x) | Je 5 Min. |
| 6 | Behandeln mit 10-fachem Volumen wässriger 10 Gew.-% Natriumchloridlösung | 20 Std. |
| 7 | Spülen mit H₂O (3x) | Je 5 Min. |
| 8 | Behandeln mit 10-fachem Volumen wässriger 10 Gew.-% Natriumchloridlösung | 20 Std. |
| 9 | Spülen mit H₂O (3x) | Je 5 Min. |
| 10 | Behandeln mit 10-fachem Volumen wässriger 10 Gew.-% Natriumchloridlösung | 20 Std. |
| 11 | Spülen mit H₂O (3x) | Je 5 Min. |
| 12 | Behandeln mit 10-fachem Volumen wässriger 10 Gew.-% Natriumchloridlösung | 20 Std. |
| 13 | Spülen mit H₂O (3x) | Je 5 Min. |
| 14 | Behandeln mit 10-fachem Volumen wässriger 10 Gew.-% Natriumchloridlösung | 20 Std. |
| 15 | Spülen mit H₂O (3x) | Je 5 Min. |
| 16 | Behandeln mit 10-fachem Volumen wässriger 10 Gew.-% Natriumchloridlösung | 20 Std. |
| 17 | Spülen mit H₂O (3x) | Je 5 Min. |
| 18 | Behandeln mit 10-fachem Volumen wässriger 3 Gew.-% Wasserstoffperoxidlösung | 24 Std. |
| 19 | Behandeln mit 10-fachem Volumen wässriger 3 Gew.-% Wasserstoffperoxidlösung | 72 Std. |
| 20 | Spülen mit H₂O (3x) | Je 5 Min. |
| 21 | Spülen mit wässriger 0,9 Gew.-% Natriumchloridlösung (3x) | Je 15 Min. |
| 22 | Spülen mit H₂O (3x) | Je 5 Min. |
| 23 | Behandeln mit 10-fachem Volumen Aceton | 16 Std. |
| 24 | Behandeln mit 10-fachem Volumen Aceton | 24 Std. |
| 25 | Behandeln mit 10-fachem Volumen Aceton | 24 Std. |
| 26 | Behandeln mit 10-fachem Volumen Aceton | 24 Std. |
| 27 | Behandeln mit 10-fachem Volumen Aceton | 24 Std. |
| 28 | Behandeln mit 10-fachem Volumen Aceton | 24 Std. |
| 29 | Behandeln mit 10-fachem Volumen Aceton | 24 Std. |
| 30 | Umlufttrocknung | 72 Std. |

Als Wasser wurde in allen Lösungen destilliertes Wasser bzw. Wasser mit einem entsprechenden Reinheitsgrad eingesetzt.

Nach der chemischen Behandlung wurde von den Rippenknorpelstücken jeweils die Knorpelhaut entfernt. Danach wurden die die porcinen Rippenknorpelstücke auf die gewünschten Ausmaße geschnitten und schließlich jeweils in 1,5 Gew.-% Wasserstoffperoxid und 0,9 Gew.-% Natriumchlorid enthaltenden wässrigen Lösungen rehydratisiert und gelagert, wobei das Wasserstoffperoxid dem Zweck dient, ein etwaiges Keimwachstum in dem Knorpelgewebe zu verhindern. Anschließend wurden die einzelnen, in Flüssigkeit gelagerten Rippenknorpelstücke verpackt und zwecks Sterilisierung bestrahlt, und zwar mit gamma-Strahlen mit einer Dosis von 20 kGy.

Die so hergestellten Transplantate wiesen eine der nativen Struktur nahezu identische Morphologie auf.

## Patentansprüche

1. Verfahren zur Konservierung von tierischem Knorpelgewebe, insbesondere von porcinem Knorpelgewebe, welches die nachfolgenden Schritte umfasst:
a) Bereitstellen von tierischem Knorpelgewebe,
b) ein- oder mehrmaliges Behandeln des tierisches Knorpelgewebes mit einer in Bezug auf die Knorpelgewebezellen hypertonen Lösung von einem oder mehreren Alkalimetallhalogenidsalzen,
c) ein- oder mehrmaliges Behandeln des tierisches Knorpelgewebes mit einem Oxidationsmittel ausgewählt aus der Gruppe bestehend aus Peroxiden, Hypochloriten, Percarbonsäuren, Ozon und beliebigen Mischungen hiervon sowie
d) ein- oder mehrmaliges Behandeln des tierisches Knorpelgewebes mit einem Lösungsmittel, welches mit Wasser bei 20°C vollständig mischbar ist,
wobei das tierische Knorpelgewebe während des Verfahrens nicht mit einem alkalischen Medium mit einem pH-Wert von 9 oder mehr in Kontakt gebracht wird, und wobei in dem Schritt a) porcines Knorpelgewebe bereitgestellt wird, welches aus einem Schwein mit einem Alter von maximal 2 Jahren stammt.

2. Verfahren nach Anspruch 1,
**dadurch gekennzeichnet, dass**
in dem Schritt a) porcines Rippenknorpelgewebe bereitgestellt wird.

3. Verfahren nach Anspruch 1 oder 2,
**dadurch gekennzeichnet, dass** das tierische Knorpelgewebe in dem Schritt b) mit einer wässrigen Lösung von einem oder mehreren Alkalimetallhalogeniden ausgewählt aus Lithiumchlorid, Natriumchlorid, Kaliumchlorid und beliebigen Mischungen von zwei oder mehr hiervon behandelt wird.

4. Verfahren nach Anspruch 3,
**dadurch gekennzeichnet, dass** das tierische Knorpelgewebe in dem Schritt b) mit einer wässrigen Lösung behandelt wird, welche 5 bis 20 Gew.-%, bevorzugt 5 bis 15 Gew.-% und besonders bevorzugt 8 bis 12 Gew.-% Natriumchlorid enthält.

5. Verfahren nach zumindest einem der vorstehenden Ansprüche,
**dadurch gekennzeichnet, dass** das tierische Knorpelgewebe in dem Schritt b) 1- bis 20-mal, bevorzugt 3-bis 15-mal, besonders bevorzugt 5- bis 10-mal, ganz besonders bevorzugt 7- bis 9-mal und höchst bevorzugt 8-mal mit jeweils frischer hypertoner Salzlösung behandelt wird.

6. Verfahren nach zumindest einem der vorstehenden Ansprüche,
**dadurch gekennzeichnet, dass** das tierische Knorpelgewebe in dem Schritt c) mit einer wässrigen Wasserstoffperoxidlösung behandelt wird, welche 0,5 bis 10 Gew.-%, bevorzugt 1 bis 5 Gew.-% und besonders bevorzugt 2 bis 4 Gew.-% Wasserstoffperoxid enthält.

7. Verfahren nach zumindest einem der vorstehenden Ansprüche,
**dadurch gekennzeichnet, dass** das tierische Knorpelgewebe in dem Schritt c) 1- bis 5-mal, bevorzugt 1- bis 3-mal und besonders bevorzugt 2-mal mit jeweils frischem Oxidationsmittel behandelt wird.

8. Verfahren nach zumindest einem der vorstehenden Ansprüche,
**dadurch gekennzeichnet, dass** das tierische Knorpelgewebe in dem Schritt d) mit einem Lösungsmittel behandelt wird, welches aus der Gruppe ausgewählt wird, welche aus Aceton, Methanol, Ethanol, Propanol, Isopropanol, Methylethylketon und beliebigen Mischungen aus zwei oder mehr dieser Verbindungen besteht, und bevorzugt das tierische Knorpelgewebe in dem Schritt d) mit Aceton behandelt wird.

9. Verfahren nach zumindest einem der vorstehenden Ansprüche,
**dadurch gekennzeichnet, dass** dieses desweiteren einen bevorzugt nach dem Schritt d) durchgeführten Schritt e) umfasst, bei dem das Perichondrium bevorzugt durch Fräsen entfernt wird.

10. Verfahren nach zumindest einem der vorstehenden Ansprüche,
**dadurch gekennzeichnet, dass** das tierische Knorpelgewebe während des Verfahrens nicht mit einem alkalischen Medium mit einem pH-Wert von 8 oder mehr in Kontakt gebracht wird.

11. Verfahren nach zumindest einem der vorstehenden Ansprüche,
**dadurch gekennzeichnet, dass**
dieses die folgenden Schritte umfasst:
a) Bereitstellen von porcinem Knorpelgewebe, welches aus einem Schwein mit einem Alter von maximal 2 Jahren stammt,
b) ein- oder mehrmaliges Behandeln des porcinem Knorpelgewebes mit einer wässrigen Lösung, welche 5 bis 20 Gew.-%, bevorzugt 5 bis 15 Gew.-% und besonders bevorzugt 8 bis 12 Gew.-% Natriumchlorid enthält,
c) ein- oder mehrmaliges Behandeln des tierisches Knorpelgewebes mit einer 1 bis 5 Gew.-% Wasserstoffperoxid enthaltenden wässrigen Lösung und
d) ein- oder mehrmaliges Behandeln des tierisches Knorpelgewebes mit Aceton,
wobei das tierische Knorpelgewebe während des Verfahrens nicht mit einem alkalischen Medium mit einem pH-Wert von 9 oder mehr in Kontakt gebracht wird.

## Claims

1. A method of conserving animal cartilage tissue, in particular porcine cartilage tissue, which comprises the following steps:
a) providing animal cartilage tissue;
b) treating the animal cartilage tissue once or a multiple of times with a solution of one or more alkali metal halide salts which is hypertonic with respect to the cartilage tissue cells;
c) treating the animal cartilage tissue once or a multiple of times with an oxidation means selected from the group comprising peroxides, hypochlorites, percarboxylic acids, ozone and any desired mixtures thereof; and
d) treating the animal cartilage tissue once or a multiple of times with a solvent which is completely miscible with water at 20°C,
wherein the animal cartilage tissue is not brought into contact with an alkaline medium having a pH of 9 or more during the method, and wherein, in step a), porcine cartilage tissue is provided which originates from a pig having an age of maximum two years.

2. A method in accordance with claim 1,
**characterized in that**
porcine rib cartilage tissue is provided in step a).

3. A method in accordance with claim 1 or claim 2,
**characterized in that**,
in step b), the animal cartilage tissue is treated with an aqueous solution of one or more alkali metal halides selected from lithium chloride, sodium chloride, potassium chloride and any desired mixtures of two or more thereof.

4. A method in accordance with claim 3,
**characterized in that**,
in step b), the animal cartilage tissue is treated with an aqueous solution which includes 5 to 20% by weight of sodium chloride, preferably 5 to 15% by weight of sodium chloride, and particularly preferably 8 to 12% by weight of sodium chloride.

5. A method in accordance with at least one of the preceding claims,
**characterized in that**,
in step b), the animal cartilage tissue is treated once to twenty times, preferably three to fifteen times, particularly preferably five to ten times, very particularly preferably seven to nine times, and most preferably eight times, with a fresh hypertonic salt solution in each case.

6. A method in accordance with at least one of the preceding claims,
**characterized in that**,
in step c), the animal cartilage tissue is treated with an aqueous hydrogen peroxide solution which includes 0.5 to 10% by weight of hydrogen peroxide, preferably 1 to 5% by weight of hydrogen peroxide, and particularly preferably 2 to 4% by weight of hydrogen peroxide.

7. A method in accordance with at least one of the preceding claims,
**characterized in that**,
in step c), the animal cartilage tissue is treated once to five times, preferably once to three times, and particularly preferably twice, with fresh oxidation means in each case.

8. A method in accordance with at least one of the preceding claims,
**characterized in that**,
in step d), the animal cartilage tissue is treated with a solvent which is selected from the group comprising acetone, methanol, ethanol, propanol, isopropanol, methyl ethyl ketone and any desired mixtures of two or more of these compounds; and **in that** the animal cartilage tissue is preferably treated with acetone in step d).

9. A method in accordance with at least one of the preceding claims,
**characterized in that**
it furthermore comprises a step e) which is preferably carried out after step d) and in which the perichondrium is preferably removed by milling.

10. A method in accordance with at least one of the preceding claims,
**characterized in that**
the animal cartilage tissue is not brought into contact with an alkaline medium having a pH of 8 or more during the method.

11. A method in accordance with at least one of the preceding claims,
**characterized in that**
it comprises the following steps:
a) providing porcine cartilage tissue which originates from a pig having an age of maximum two years;
b) treating the porcine cartilage tissue once or a multiple of times with an aqueous solution which includes 5 to 20% by weight of sodium chloride, preferably 5 to 15% by weight of sodium chloride, and particularly preferably 8 to 12% by weight of sodium chloride;
c) treating the animal cartilage tissue once or a multiple of times with an aqueous solution including 1 to 5% by weight of hydrogen peroxide; and
d) treating the animal cartilage tissue once or a multiple of times with acetone,
wherein the animal cartilage tissue is not brought into contact with an alkaline medium having a pH of 9 or more during the method.

## Revendications

1. Procédé pour conserver du tissu cartilagineux animal, en particulier du tissu cartilagineux porcin, comprenant les étapes suivantes consistant à :
a) approvisionner du tissu cartilagineux animal,
b) traiter une ou plusieurs fois le tissu cartilagineux animal avec une solution, hypertonique par rapport aux cellules du tissu cartilagineux, d'un ou de plusieurs sels d'halogénure de métal alcalin,
c) traiter une ou plusieurs fois le tissu cartilagineux animal avec un agent oxydant choisi parmi le groupe comprenant les peroxydes, les hypochlorites, les acides percarboxyliques, l'ozone et des mélanges quelconques de ceux-ci, et
d) traiter une ou plusieurs fois le tissu cartilagineux animal avec une solution qui est complètement miscible avec de l'eau à 20 °C,
dans lequel
pendant le procédé, le tissu cartilagineux animal n'est pas mis en contact avec un milieu alcalin d'un pH de 9 ou plus, et
dans l'étape a), du tissu cartilagineux porcin est approvisionné qui est issu d'un porc d'un âge maximal de 2 ans.

2. Procédé selon la revendication 1,
**caractérisé en ce que**
dans l'étape a), un tissu cartilagineux porcin des côtes est approvisionné.

3. Procédé selon la revendication 1 ou 2,
**caractérisé en ce que**
dans l'étape b), le tissu cartilagineux animal est traité avec une solution aqueuse d'un ou de plusieurs halogénures de métal alcalin choisis parmi le chlorure de lithium, le chlorure de sodium, le chlorure de potassium et des mélanges quelconques de deux ou de plusieurs de ceux-ci.

4. Procédé selon la revendication 3,
**caractérisé en ce que**
dans l'étape b), le tissu cartilagineux animal est traité avec une solution aqueuse qui contient 5 à 20 % en poids, de préférence 5 à 15 % en poids et de préférence particulière 8 à 12 % en poids de chlorure de sodium.

5. Procédé selon l'une au moins des revendications précédentes,
**caractérisé en ce que**
dans l'étape b), le tissu cartilagineux animal est traité 1 à 20 fois, de préférence 3 à 15 fois, de préférence particulière 5 à 10 fois, de façon tout particulièrement préférée 7 à 9 fois et de préférence absolue 8 fois avec une solution saline hypertonique respectivement fraîche.

6. Procédé selon l'une au moins des revendications précédentes,
**caractérisé en ce que**
dans l'étape c), le tissu cartilagineux animal est traité avec une solution aqueuse de peroxyde d'hydrogène qui contient 0,5 à 10 % en poids, de préférence 1 à 5 % en poids, et de préférence particulière 2 à 4 % en poids de peroxyde d'hydrogène.

7. Procédé selon l'une au moins des revendications précédentes,
**caractérisé en ce que**
dans l'étape c), le tissu cartilagineux animal est traité 1 à 5 fois, de préférence 1 à 3 fois et de préférence particulière 2 fois avec un agent oxydant respectivement frais.

8. Procédé selon l'une au moins des revendications précédentes,
**caractérisé en ce que**
dans l'étape d), le tissu cartilagineux animal est traité avec un solvant qui est choisi parmi le groupe comprenant l'acétone, le méthanol, l'éthanol, le propanol, l'isopropanol, le méthlyéthylcétone et des mélanges quelconques de deux de ces composés ou plus, et de préférence, dans l'étape d), le tissu cartilagineux animal est traité avec de l'acétone.

9. Procédé selon l'une au moins des revendications précédentes,
**caractérisé en ce que**
celui-ci comprend en outre une étape e) effectuée de préférence après l'étape d), dans laquelle le périchondre est enlevé de préférence par fraisage.

10. Procédé selon l'une au moins des revendications précédentes,
**caractérisé en ce que**
pendant le procédé, le tissu cartilagineux animal n'est pas mis en contact avec un milieu alcalin d'un pH de 8 ou de plus.

11. Procédé selon l'une des revendications précédentes,
**caractérisé en ce que**
celui-ci comprend les étapes suivantes consistant à :
a) approvisionner un tissu cartilagineux porcin qui est issu d'un porc d'un âge maximal de 2 ans,
b) traiter une ou plusieurs fois le tissu cartilagineux porcin avec une solution aqueuse qui contient 5 à 20 % en poids, de préférence 5 à 15 % en poids et de préférence particulière 8 à 12 % en poids de chlorure de sodium,
c) traiter une ou plusieurs fois le tissu cartilagineux animal avec une solution aqueuse qui contient 1 à 5 % en poids de peroxyde d'hydrogène,
d) traiter une ou plusieurs fois le tissu cartilagineux animal avec de l'acétone,
dans lequel, pendant le procédé, le tissu cartilagineux animal n'est pas mis en contact avec un milieu alcalin d'un pH de 9 ou plus.
